# EUROPÄISCHE PATENTSCHRIFT

(11) **EP 2 474 830 B1**
(45) Veröffentlichungstag und Bekanntmachung des Hinweises auf die Patenterteilung: **24.09.2014**
(21) Anmeldenummer: 12160723.8
(22) Anmeldetag: 06.02.2006
(51) Int. Cl.: G01N 33/86, C12Q 1/56, G01N 33/49

(54) **Methode zur Feststellung von von Faktor XIII-Mangelzuständen unter Einsatz thrombelastographischer Techniken**
Method for determining Factor XIII deficiencies using thromboelastographic techniques
Méthode de détermination du niveau de déficience en facteur XIII en utilisant des techniques de thrombo-élastographie

(30) Priorität: 08.02.2005 DE 102005005824
(43) Veröffentlichungstag der Anmeldung: 11.07.2012
(62) Teilanmeldung aus: 06706670.4
(73) Patentinhaber: CSL Behring GmbH, 35041 Marburg (DE)
(72) Erfinder: Wrabetz, Erhardt, 65239 Hochheim (DE); Metzner, Hubert, 35041 Marburg (DE); Korte, Wolfgang, 9010 St. Gallen (CH)
(74) Vertreter: Hauser, Hans-Peter

(56) Entgegenhaltungen:
- NIELSEN VANCE G ET AL: "The impact of factor XIII on coagulation kinetics and clot strength determined by thrombelastography", ANESTHESIA & ANALGESIA, Bd. 99, Nr. 1, Juli 2004 (2004-07), Seiten 120-123, XP009066431, ISSN: 0003-2999
- ESTNER M E ED - ESTNER M E: "Effekte von Faktor XIII auf die Gerinnselbildung und Gerinnselfestigkeit vor und nach herzchirurgischen Eingriffen", 1. Januar 2001 (2001-01-01), EFFEKTE VON FAKTOR XIII AUF DIE GERINNSELBILDUNG UND GERINNSELFESTIGKEIT VOR UND NACH HERZCHIRURGISCHEN EINGRIFFEN, KLINIK FUR ANAESTHESIOLOGIE DER TECHNISCHEN UNIVERSITAT MUNCHEN KLINIKUM RECHTS DER ISAR, PAGE(S) I - VIII,1, XP003028133, * das ganze Dokument, insbesondere die Seiten 6 und 43-44 *
- TYLER H M: "Fibrin crosslinking demonstrated by thrombelastography", THROMBOSIS ET DIATHESIS HAEMORRHAGICA, SCHATTAUER GMBH, DE, Bd. 22, Nr. 2, 15. November 1969 (1969-11-15), Seiten 398-400, XP003028134, ISSN: 0340-5338
- WILMER M ET AL: "Methoden zur Bestimmung des Faktors XIII/XIIIa // Methods for the determination of factor XIII/XIIIa", HAEMOSTASEOLOGIE, STUTTGART, DE, Bd. 22, Nr. 1, 1. Januar 2002 (2002-01-01) , Seiten 18-28, XP009133425, ISSN: 0720-9355
- NIELSEN V G ET AL: "Effects of coagulation factor deficiency on plasma coagulation kinetics determined via thrombelastography(R): critical roles of fibrinogen and factors II, VII, X and XII", ACTA ANAESTHESIOLOGICA SCANDINAVICA, Bd. 49, Nr. 2, Februar 2005 (2005-02), Seiten 222-231, XP002383695, ISSN: 0001-5172

## Beschreibung

### 1. Einleitung

Die Erfindung betrifft eine Methode zur Feststellung von Faktor XIII Mangelzuständen durch Messung, Auswertung und Vergleich von einem oder mehreren der TEG Parameter Reaktionszeit (r), der maximalen Amplitude (MA), der Zeit bis zum Erreichen der maximalen Amplitude (TMA) und des Winkels Alpha in Anwesenheit und Abwesenheit von Faktor XIII Inhibitoren.

### 2. Darstellung des Standes der Technik

Die Thrombelastographie (TEG) ist eine diagnostische Methode, die mechanisch die Gerinnselentstehung oder -Auflösung in einem oszillierenden System untersucht. Dabei wird entweder ein Gefäß (Cup) oszillierend um einen Messstab (Pin) bewegt (herkömmliche TEG) oder aber das Gefäß steht fest und der Pin wird in oszillierende Rotationsbewegungen gebracht (ROTEG bzw. ROTEM). Die zwischen Cup und Pin auftretenden mechanischen Kräfte werden aufgezeichnet. Sobald das Blut oder Plasma zur Gerinnung kommt, tritt eine Abweichung zum anfänglichen Messsignal auf. Beide Ausführungen sollen hier als TEG bezeichnet werden.

Die TEG wird zur Untersuchung von Blut oder Plasma eingesetzt, um gerinnungsrelevante Parameter (TEG Parameter), wie die Zeitspanne bis zum Erreichen einer ersten signifikanten Clot-Bildung mit einer Amplitude von 2mm (Gerinnungs- oder Reaktionszeit r), die Zeitspanne bis zum Erreichen einer Clot-Stärke von einer Amplitude von 20 mm (k-Wert), die Geschwindigkeit, mit der das Gerinnsel gebildet wird (Winkel Alpha), die mechanischen Eigenschaften des Gerinnsels bei maximaler Amplitude (MA) oder zu einem anderen beliebigen Zeitpunkt, die Zeitspanne bis zur MA (TMA), oder die Zeitspanne bis die Gerinnselfestigkeit aufgrund von Fibrinolyse auf einen bestimmten Wert wieder abgefallen ist, zu bestimmen. Klinisch wird die TEG u.a. zur Beurteilung der Koagulopathie beispielsweise in der Herzchirurgie, bei Lebertransplantationen, großer Abdominalchirurgie und als quasi Bedside-Test im perioperativen Gerinnungsmanagement als diagnostische Maßnahme eingesetzt (Kettner SC et al. (1999), Anesth Analg; 89: 580-584; Shore-Lesserson L et al. (1999), Anesth Analg; 88: 312-319et al.; Harding SA et al. (1997), Br J Anaesth; 78: 175-179; Kettner SC et al. (1998), Anesth Analg; 86: 691-695, Pivalizza EG et al. (1998), J Cardiothorac Vasc Anesth; 12: 305-308., Mahla E et al. (2001), Anest Analg; 92: 572-577; Calatzis AN et al. (1996), Eur Surg Res; 28: S1 (89), Mahla E et al. (2001), Anesth Analg; 92 (3): 572-577).

Die TEG steht ergänzend zur Standard Gerinnungsdiagnostik (u.a. Quick, aPTT, Thrombozytenzahl, AT III, Fibrinogen, D-Dimere, Blutungszeit), die zur Ermittlung der Werte zeitaufwendiger ist, für eine schnelle Orientierung über Blutungstendenzen zur Verfügung. Dies ist besonders dann von Bedeutung, wenn Koagulopathien im Rahmen von extensiven chirurgischen Eingriffen oder nach Polytraumata auftreten, da schwere Störungen der Hämostase rasch zur Entwicklung von sekundären Gewebeschäden führen können und oft resistent sind gegenüber einer konventionellen hämostatischen Therapie auf der Basis einer zeitaufwendigeren Standard-Gerinnungsdiagnostik.

Die genannten TEG Parameter werden durch eine Reihe von Faktoren beeinflusst, die im Folgenden als thrombelastographisch relevante Faktoren bezeichnet werden. Thrombelastographisch relevante Faktoren sind vor allem Fibrinogen-, Faktor XIII-und Blutplättchen-Spiegel sowie Komponenten des fibrinolytischen Systems wie Plasmin, Plasmin-Aktivatoren und Plasmin-Inhibitoren. So korreliert beispielsweise Fibrinogen als Substrat der Gerinnung mit der Clot-Stabilität. Dies kann im Thrombelastogramm eindeutig gezeigt werden. Darüber hinaus moduliert der Faktor XIII über die Vernetzung des aus dem Fibrinogen gebildeten Fibrins ebenfalls die mechanischen Eigenschaften des Clots und schränkt dessen Lyse ein (P. Lauer et al. (2002), Thromb Haemost; 88:967-974). Beide Effekte sind in der Thrombelastographie bislang nicht eindeutig voneinander trennbar, da sie zentrale Messgrößen eines Thrombelastogrammes gleichermaßen beeinflussen. Sowohl Fibrinogen als auch Faktor XIII beeinflussen die Parameter Gerinnungszeit (r), maximale Clot-Festigkeit, Winkel Alpha als Maß für die Geschwindigkeit der Clot-Bildung und die Lysezeit des Clots signifikant (Nielsen VG et al. (2004), Anesth Analg; 99: 120-123). Da bei erworbenen Mangelzuständen, beispielsweise bei Polytraumata oder größeren chirurgischen Eingriffen der Fibrinogen- und der Faktor XIII-Spiegel nicht notwendigerweise gleichermaßen abfallen, ist bislang eine gezielte therapeutische Intervention nicht eindeutig zugunsten der einen oder anderen Komponente auf Basis des Thrombelastogramms möglich. Es wäre daher wünschenswert, wenn eine Methode zur Verfügung stünde, die es erlauben würde, auf Basis der schnellen TEG Diagnostik eine Aussage darüber treffen zu können, ob eine Blutungsneigung auf einem Faktor XIII- und/oder auf einem Fibrinogen-Mangel beruht.

WO2004/092742 beschreibt ein Verfahren zur Messung eines Thrombinmangels. Dabei wird den Proben Heparin als indirektes Antithrombin Antikoagulant zugegeben.

WO0107070 offenbart ein Verfahren zur Messung der Aktivität von Gerinnungsfaktoren, wobei den Proben Inhibitoren gegen verschiedene Gerinnungsfaktoren zugegeben werden und die Gerinnungszeit gemessen wird. Die Methode dient dem Auffinden von hyperkoagulativen Zuständen.

WO2004081579 offenbart die Verwendung der Thrombelastographie zur Verfolgung verschiedener thrombelastographischer Parameter bei der Plättchenhemmung.

US 6,245,573 befasst sich mit dem Einfluss von Metallionen auf die Viskosität und die Gerinnungsaktivität von Blut

Craft et al. (American Society of Anesthesiologists, Philadelphia, PA, US Nr. 2003, 15.Okt. 2003, Abstract Nr. 148)

Katori et al. (Anesthesia and Analgesia, Bd. 99, Nr. 6 (2004) beschreibt ein mittels Batroxobin modifizierte Thrombelastographie zum Nachweis einer Eptifibatide induzierten Plättchenhemmung.

Nielsen et al. (Acta Anaeasthesiologica Scandinavia, Bd. 49, Nr. 2 Seiten 222-231 (2005) befasst sich mit der Rolle von Fibrinogen bei thrombelastographischen Messungen. Es zeigt sich eine klare Abhängigkeit der Thrombelastographie von der Fibrinogen Konzentration.

Estner (Dissertation eingereicht am 23.4.2001 bei der Technischen Universität München) untersucht die Rolle von Faktor XIII auf die Gerinnselbildung und Gerinnselfestigkeit vor und nach herzchirurgischen Eingriffen mit Hilfe der Thrombelastographie.

Wilmer et al. (Hämatoserologie 1, (2002)) beschreibt die Verwendung verschiedener Methoden, darunter auch der Thrombelastographie für die Bestimmung der Gerinnselbildung und der Gerinnselqualität.

Tyler et al. ((1969) untersucht den Einfluss der Quervernetzung von Fibrin auf thrombelastographische Parameter.

### 3. Darstellung von Aufgabe und Lösung

Den vorliegenden Untersuchungen lag die Aufgabe zu Grunde, mittels thrombelastographischer Techniken eine Bestimmung des Gehalts an Faktor XIII zu ermöglichen, um dadurch eine gezielte therapeutische Intervention durch Substitution des fehlenden Faktors zu ermöglichen.

Gelöst wurde die Aufgabe dadurch, dass ein Verfahren entwickelt wurde, um Faktor XIII Mangelzustände durch Messung, Auswertung und Vergleich von einem oder mehreren der TEG Parameter Reaktionszeit (r), der maximalen Amplitude (MA), der Zeit bis zum Erreichen der maximalen Amplitude (TMA) und des Winkels Alpha in Anwesenheit und Abwesenheit von Faktor XIII Inhibitoren zu bestimmen.

### 4. Detaillierte Beschreibung der Erfindung und verschiedener Ausführungsformen

Durch Vergleich der TEG Parameter, wie beispielsweise der Reaktionszeit (r), der maximalen Amplitude (MA), der Zeit bis zum Erreichen der maximalen Amplitude (TMA) oder des Winkels Alpha in An- oder Abwesenheit von Faktor XIII Inhibitoren, kann man zwischen einem Faktor XIII-Mangel und anderen Mangelzuständen als Ursache für Hämostasestörungen differenzieren.

Zur Hemmung des Faktor XIII können beispielsweise Antikörper gegen Faktor XIII, peptidische Inhibitoren wie Tridegin (Finney S. et al. (1997), Biochem J. 324: 797-805) oder niedermolekulare Inhibitoren des Faktor XIII, wie beispielsweise Putrescin, Dansylcadaverin o.a. (Prasa D. et al. (2002), Hämostaseologie 22: 29-33) verwendet werden. Der Faktor XIII Inhibitor bzw. die Konzentration des Faktor XIII Inhibitors ist bevorzugt so zu wählen, dass die Faktor XIII Aktivität in der untersuchten Probe spezifisch und vollständig inhibiert wird.

Je geringer der Faktor XIII-Spiegel bei einem Patienten ist, desto geringer wird dabei der Unterschied zwischen dem Wert eines TEG Parameters, den man in Anwesenheit und dem, den man in Abwesenheit eines Faktor XIII-Inhibitors misst. Die Auswertung der Differenz der TEG Parameter in An- und Abwesenheit eines Faktor XIII Inhibitors lässt darauf schließen, ob ein leichter, mittlerer oder schwerer Faktor XIII Mangel vorliegt. Durch Aufstocken einer von einem Patienten mit Faktor XIII Defizienz gewonnenen Vollblutprobe mit unterschiedlichen Mengen an Faktor XIII und durch Vergleich des Thrombelastogramms in An- und Abwesenheit eines Inhibitors von Faktor XIII in diesen Proben lässt sich der Effekt auch in Form einer Standardkurve darstellen. Eine solchermaßen gewonnene Kurve erlaubt es, aus der Differenz eines TEG Parameters in An- und Abwesenheit eines Faktor XIII-Inhibitors den Faktor XIII-Spiegel in einer Patientenprobe zu bestimmen. Bevorzugt werden entsprechende Standardkurven bei unterschiedlichen Fibrinogen-Gehalten erstellt.

Durch Bezug der Differenz der Patientenprobe in An- und Abwesenheit eines Faktor XIII Inhibitors auf das Verhältnis des TEG Parameters (mit Inhibitorzusatz) des Patientenplasmas zu einer Kontrollprobe ergibt sich auch die Möglichkeit, die Messdifferenz zu relativieren und somit unterschiedliche Fibrinogenspiegel zu berücksichtigen, z.B. durch Bildung des folgenden Terms im Falle der maximalen Amplitude: (MA_{Probe ohne Inh.} - MA_{Probe} mit _{Inh.}) MA_{Kontrolle} mit _{Inh} / MA_{Probe} mit _{Inh}.).

Analoge Standardkurven können auch mit plättchenarmem und plättchenreichem Plasma erhalten werden. Unterhalb eines Wertes von 70% des Normalwerts von Faktor XIII in Plasma spricht man von Faktor XIII Mangelzuständen, die therapiert werden sollten (T. Muto et al. (1997), Biomed. Progress 10: 16-19).

Gegenstand der Erfindung ist daher eine Methode zur Feststellung von Faktor XIII Mangelzuständen durch Messung, Auswertung und Vergleich von einem oder mehreren der TEG Parameter Reaktionszeit (r), der maximalen Amplitude (MA), der Zeit bis zum Erreichen der maximalen Amplitude (TMA) und des Winkels Alpha in Anwesenheit und Abwesenheit von Faktor XIII Inhibitoren.

### 5. Beispiele

### 1. Hemmung von Faktor XIII in Plasma durch Zusatz von Faktor XIII-Antikörpern

Um den Einfluss von Faktor XIII quantitativ zu ermitteln, wurde in einem Experiment Standardhumanplasma ohne bzw. mit Zusatz von Faktor XIII Inhibitoren (Anti-Faktor XIII IgG Präparation) mittels TEG analysiert. Die Gerinnungsreaktion wurde durch Zusatz eines aPTT Reagenzes beschleunigt. Der Testansatz enthielt NaCl-Lsg. oder anti-Faktor XIII IgG Präparation in unterschiedlichen Verdünnungen (30 µL), Pathromtin SL (50 µL, Dade Behring), Standard Humanplasma (200 µL) und 200 mM CaCl2-Lsg (20 µL). Bei 37°C wurden die Reagenzien in das Cup pipettiert und die TEG Messung an Geräten der Firma Haemoscope gestartet. Folgende Messparameter wurden ausgewertet: R, Winkel alpha, Maximalamplitude und Zeit bis zum Erreichen der Maximalamplitude.

**Tabelle 1: Hemmung von Faktor XIII in Plasma durch Zusatz von Faktor-XIII Antikörpern**

| | | **R (sec)** | **Angle (°)** | **MA (mm)** | **TMA (sec)** | |
|---|---|---|---|---|---|---|
| **Kontrolle (ohne F XIII AK)** | | **133,8** | **70,7** | **16,7** | **721,3** | Mittelwerte aus n=8 |
| | | | | | | |
| **Anti FXIII A IgG Präp.** | **1:3** | **217,5** | **53,3** | **7,1** | **272,5** | n=2 |
| | **1:10** | **157,5** | **61,7** | **9,9** | **382,5** | n=2 |
| | **1:30** | **152,5** | **67,5** | **15,1** | **605,0** | n=2 |
| | **1:100** | **120,0** | **69,2** | **16,3** | **687,5** | n=2 |

Die Ergebnisse zeigen, dass sich die Faktor XIII-Inhibition deutlich auf die gemessenen TEG Parameter auswirkt. Entsprechend würde ein Vergleichsansatz (+/-Faktor XIII Inhibitor) bei einer Patientenprobe mit reduzierter maximaler Amplitude eine Abschätzung ermöglichen, ob noch eine ausreichende Faktor XIII-Menge in der Probe vorhandenen ist oder ob diese signifikant reduziert ist (bei reduziertem Faktor XIII-Spiegel würde der Effekt der Faktor XIII-Inhibitor Zugabe geringer ausfallen als bei normalem Faktor XIII-Spiegel).

### 2. Untersuchung von Plasmen und Vollblut durch parallele Messungen in An-und Abwesenheit von niedermolekularen Faktor XIII-Inhibitoren

Zur quantitativen Bestimmung des Einflusses von Faktor XIII wurde Standardhumanplasma ohne bzw. mit Zusatz niedermolekularer Faktor XIII Inhibitoren mittels TEG analysiert. Die Gerinnungsreaktion wurde durch Zusatz eines Gewebefaktorreagenzes gestartet bzw. beschleunigt. Der Testansatz enthielt: 200 µL Plasma, 30 µL NaCl-Lsg. (Kontrolle) oder Inhibitor-Lösung in unterschiedlichen Verdünnungen, 50 µL Gewebefaktorreagenz (Thromborel S, Dade Behring) und 20 µL Calciumchlorid-Lösung (200 mMol/L). Bei 37°C wurden die Reagenzien in das Cup pipettiert und die TEG Messung an Geräten der Firma Haemoscope gestartet. Alternativ kann das Calciumchlorid auch bereits zu dem Gewebefaktorreagenz zugegeben und damit die Reaktion gestartet werden. Die Messparameter Reaktionszeit (R), Winkel alpha, Maximalamplitude (MA) und Zeit bis zum Erreichen der Maximalamplitude (TMA) wurden erfasst und ausgewertet.
Als F XIII Inhibitoren zur Differenzierung des F XIII Gehaltes wurden Putrescin, Histidin, Dansylcadaverin bzw. 1,3,4,5-Tetramethyl-2-[(2-oxopropyl)thio]-imidazoliumchlorid in unterschiedlichen Verdünnungen eingesetzt. Die hier mit Plasma durchgeführten Untersuchungen sind prinzipiell auch auf Vollblut übertragbar.

**Tabelle 2: Effekt von Putrescin, Monodansylcadaverin und Histamin auf die TEG Parameter bei Verwendung von Standardhumanplasma.**

| **Putrescin** | **R:** | **Angle:** | **MA:** | **TMA:** |
|---|---|---|---|---|
| Konzentration im Test: (µg/mL) | **sec** | **°** | **mm** | **sec** |
| **1,07** | **22,5** | **73,1** | **17,2** | **452,5** |
| **3,21** | **17,5** | **73,7** | **15,9** | **277,5** |
| **10,7** | **20,0** | **73,4** | **18,7** | **672,5** |
| **32,1** | **25** | **73,0** | **16,9** | **585,0** |
| **107** | **15** | **73.0** | **16,5** | **505,0** |
| **321** | **20** | **72,8** | **15,8** | **400,0** |
| **1.071** | **25** | **69,5** | **13,6** | **405,0** |
| **3.214** | **32,5** | **72,6** | **13,9** | **327,5** |
| | | | | |

| **Monodansylcadaverin** | **R:** | **Angle:** | **MA:** | **TMA:** |
|---|---|---|---|---|
| Konzentration im Test: (µg/mL) | **sec** | **°** | **mm** | **sec** |
| **0,32** | **20,0** | **74,0** | **18,1** | **655,0** |
| **1,07** | **22,5** | **74,9** | **18,4** | **610,0** |
| **3,21** | **20,0** | **74,7** | **19,1** | **657,5** |
| **10,7** | **25,0** | **74,4** | **18,4** | **560,0** |
| **32,1** | **17,5** | **74,4** | **17,3** | **562,5** |
| **107** | **32,5** | **76,9** | **18,7** | **237,5** |
| **321** | **20,0** | **71,0** | **15,1** | **490,0** |
| **536** | **27,5** | **70,3** | **13,3** | **370,0** |
| | | | | |

| **Histamin** | **R:** | **Angle:** | **MA:** | **TMA:** |
|---|---|---|---|---|
| Konzentration im Test: (µg/mL) | **sec** | **°** | **mm** | **sec** |
| **1,07** | **20,0** | **73.4** | **17,8** | **592,5** |
| **3,21** | **22,5** | **75,0** | **17,9** | **527,5** |
| **10,7** | **20,0** | **74,8** | **19,2** | **622,5** |
| **32,1** | **22,0** | **73,9** | **17,2** | **472,5** |
| **107** | **17,5** | **73,5** | **16,9** | **462,5** |
| **321** | **20,0** | **72,7** | **15,5** | **430,0** |
| **1.071** | **15,0** | **70,3** | **14,0** | **492,5** |
| **3.214** | **25,0** | **71,9** | **13,0** | **182,5** |
| | | | | |

| **Kontrolle** | **R:** | **Angle:** | **MA** | **TMA:** |
|---|---|---|---|---|
| **(physiol. NaCl Lösung)** | **sec** | **°** | **mm** | **sec** |
| | **25,6** | **75,6** | **19,7** | **572** |

**Tabelle 3: Effekt von 1,3,4,5-Tetramethyl-2-[(2-oxopropyl)thio]-imidazoliumchlorid auf die TEG Parameter).**

| **1,3,4,5-Tetramethyl-2-** | **R:** | **Angle:** | **MA:** | **TMA:** |
|---|---|---|---|---|
| **[(2-oxopropyl)thio]-** | | | | |
| **imidazolium chloride** | **sec** | **°** | **mm** | **sec** |
| Konzentration im Test (µg/mL) | | | | |
| **0,033** | **22,5** | **79,6** | **21,5** | **177,5** |
| **0,10** | **22,5** | **79,0** | **18,5** | **140,0** |
| **0,33** | **22,5** | **77,0** | **14,9** | **77,5** |
| **1,00** | **22,5** | **75,4** | **13,1** | **77,5** |
| **3,33** | **22,5** | **74,0** | **12,4** | **77,5** |
| | | | | |
| **Kontrolle** | | | | |
| **(physiol. NaCl Lösung)** | | | | |
| | **27,5** | **77,7** | **21,6** | **520,0** |
| | | | | |

Es zeigt sich, dass die untersuchten Inhibitoren die Wirkung von F XIII inhibieren können und die relevanten TEG-Parameter wie z.B. maximale Amplitude (MA) signifikant auf den noch verfügbaren F XIII-Gehalt ansprechen. Auf diese Art und Weise kann man F XIII defiziente Plasmen identifizieren, da solche Plasmen den Effekt der F XIII Inhibitoren nicht oder nur eingeschränkt zeigen. Aus den obigen Untersuchungen ergeben sich als bevorzugte Konzentrationen an Inhibitoren diejenigen, bei denen die durch F XIII beeinflussten Parameter wie die maximale Amplitude MA nicht mehr deutlich abfallen oder ansteigen. Für 1,3,4,5-Tetramethyl-2-[(2-oxopropyl)thio]-imidazoliumchlorid ist dies z.B. eine Endkonzentration von 1 µg/mL, besonders bevorzugt von ca. 3 µg/mL oder höher.

## Patentansprüche

1. Thromboelastographische Methode zur Feststellung von Faktor XIII Mangelzuständen durch Messung, Auswertung und Vergleich von einem oder mehreren der TEG Parameter Reaktionszeit (r), der maximalen Amplitude (MA), der Zeit bis zum Erreichen der maximalen Amplitude (TMA) und des Winkels Alpha in Anwesenheit und Abwesenheit von Faktor XIII Inhibitoren.

2. Methode entsprechend Anspruch 1, wobei als Faktor XIII-Inhibitoren poly- oder monoklonale Antikörper eingesetzt werden.

3. Methode entsprechend Anspruch 1, wobei als Faktor XIII-Inhibitoren peptidische Inhibitoren eingesetzt werden.

4. Methode entsprechend Anspruch 1, wobei als Faktor XIII-Inhibitoren niedermolekulare Inhibitoren eingesetzt werden.

## Claims

1. Thromboelastographic method for the determination of factor XIII deficiency states by measuring, evaluating and comparing one or a plurality of the TEG parameters, reaction time (r), maximum amplitude (MA), time until the maximum amplitude is attained (TMA) and the angle alpha in the presence and in the absence of factor XIII inhibitors.

2. Method according to Claim 1, where polyclonal or monoclonal antibodies are employed as factor XIII inhibitors.

3. Method according to Claim 1, where peptidic inhibitors are employed as factor XIII inhibitors.

4. Method according to Claim 1, where low-molecular-weight inhibitors are employed as factor XIII inhibitors.

## Revendications

1. Procédé de thrombo-élastographie pour la détermination d'états de déficit en facteur XIII par mesure, évaluation et comparaison d'un ou plusieurs des paramètres TEG, temps de réaction (r), amplitude maximale (MA), temps nécessaire pour atteindre l'amplitude maximate (TMA) et angle alpha en présence et en l'absence d'inhibiteurs du facteur XIII.

2. Procédé conforme à la revendication 1, dans lequel on utilise comme inhibiteurs du facteur XIII des anticorps monoclonaux ou polyclonaux.

3. Procédé conforme à la revendication 1, dans lequel on utilise comme inhibiteurs du facteur XIII des inhibiteurs peptidiques.

4. Procédé conforme à la revendication 1, dans lequel on utilise comme inhibiteurs du facteur XIII des inhibiteurs de faible masse moléculaire.
